# EUROPÄISCHE PATENTANMELDUNG

(11) **EP 2 620 155 A1**
(43) Veröffentlichungstag der Anmeldung: **31.07.2013**
(21) Anmeldenummer: 12153060.4
(22) Anmeldetag: 30.01.2012
(51) Int. Cl.: A61K 36/71, A61P 21/00

(54) **Arzneimittel enthaltend Cimicifuga zur Anwendung bei der Behandlung von Sarkopenie und Herzmuskelschwäche**

(71) Anmelder: Bionorica SE, 92318 Neumarkt (DE)
(72) Erfinder: Wuttke, Wolfgang., 37120 Bovenden (DE); Seidlová-Wuttke, Dana., 37120 Bovenden (DE)
(74) Vertreter: Simandi, Claus

(57) **Zusammenfassung**

Die Erfindung betrifft Arzneimittel enthaltend Cimicifuga, Extrakte aus Cimicifuga, Saponin-haltige Fraktion aus Cimicifuga zur Anwendung bei Muskelschwäche und zur Vorbeugung gegen Muskelschwäche, insbesondere Sarkopenie und Herzmuskelschwäche bei Mensch und Tier. Die Erfindung betrifft weiterhin Zusammensetzungen, insbesondere pharmazeutische Zusammensetzungen, Nahrungsmittel, Nahrungsergänzungsmittel enthaltend Cimicifuga, Extrakte aus Cimicifuga, Saponin-haltige Fraktion aus Cimicifuga sowie Verfahren zu deren Herstellung.

## Beschreibung

Die Erfindung betrifft Arzneimittel enthaltend Cimicifuga, Extrakte aus Cimicifuga, Saponin-haltige Fraktion aus Cimicifuga zur Anwendung bei Muskelschwäche und zur Vorbeugung gegen Muskelschwäche, insbesondere Sarkopenie und Herzmuskelschwäche bei Mensch und Tier. Die Erfindung betrifft weiterhin Zusammensetzungen, insbesondere pharmazeutische Zusammensetzungen, Nahrungsmittel, Nahrungsergänzungsmittel enthaltend Cimicifuga, Extrakte aus Cimicifuga, Saponin-haltige Fraktion aus Cimicifuga sowie Verfahren zu deren Herstellung.

Cimicifuga-Extrakte sind zur Behandlung von Krankheiten auf der Basis von wäßrig-ethanolischen Auszügen bereits bekannt. Insbesondere deren Trockenextrakte werden als Phytopharmaka eingesetzt.

In den vergangenen Jahren war der Einsatz der Hormonersatztherapie (HRT) bei klimakterischen postmenopausalen Beschwerden wie Gemütsschwankungen, Hitzewallungen und vermehrtes Schwitzen weit verbreitet. Die Zunahme kardiovaskulärer und thromboembolischer Erkrankungen sowie erhöhtes Brustkrebsrisiko während der Behandlung mit HRT indizieren die Suche nach einer Medikation ohne die estrogenabhängige Nebenwirkungen. Die Anmelderin vertreibt daher das Cimicifuga-Arzneimittelprodukt Klimadynon® (Trockenextrakt aus Cimicifugawurzelstock) zur Behandlung von klimaterischen Beschwerden.

Cimicifuga-Extrakte sind als östrogenartiges organselektives Arzneimittel bereits in WO1999047149A1 beschrieben. W0200236140A offenbart, dass Zubereitungen von Cimicifuga beim Vorliegen einer Harninkontinenz therapeutisch wirksam sind. Weiterhin wird in WO1999047149A1 die Eignung von Cimicifuga-Extrakten zur Behandlung von Osteoporose und Artheriosklerose offenbart.

Es besteht jedoch ein hohes Interesse weitere Erkrankungen mittels Cimicifuga-Extrakten zu behandeln.

Die Behandlung der Sarkopenie stellt im klinischen Alltag eine Herausforderung dar, denn es stehen keine adäquaten Behandlungsmethoden zur Verfügung. Als Sarkopenie bezeichnet man den mit fortschreitendem Alter zunehmenden Muskelabbau und die damit einhergehenden funktionellen Einschränkungen von älteren Menschen. Bei den Betroffenen führt dies zu einer Häufung von Stürzen und damit verbundenen Verletzungen. Aufgrund der demographischen Entwicklung mit einer stetig wachsenden Gruppe älterer Menschen und somit immer mehr Menschen, die an Sarkopenie leiden, besteht ein großer Bedarf an Methoden und Arzneimitteln zur Behandlung und Prävention von Sarkopenie.

Zur Prävention der Sarkopenie wird Krafttraining empfohlen, wobei jedoch schnell ein Plateau erreicht wird und weiteres Training zu keinem weiteren Erfolgen führt. Durch die Gabe von Steroidhormonen lässt sich zwar die Rückbildung der Muskulatur drosseln, aber häufig treten unerwünschte Nebenwirkungen auf. Verschiedene Untersuchungen haben gezeigt, dass eine Erhöhung der Proteinaufnahme und vor allem der aufgenommenen Menge an den Aminosäuren Leucin, Isoleucin oder Valin einen positiven Effekt zeigen, beispielsweise kann über die Erhöhung der Proteinneubildung im Muskel eine Steigerung der Körpermagermasse erreicht werden (K. Norman et al. "Bedeutung der Proteinzufuhr bei der Entstehung und Behandlung der Sarkopenie" (2009) Aktuell Ernährungsmed. 34, S. 171-177.)

Koronare Herzerkrankungen, insbesondere Herzinsuffizienz (Herzmuskelschwäche), ist die zweithäufigste Todesursache in Deutschland. Medikamentös wird die Herzmuskelschwäche mit ACE-Hemmern, AT1-Antagonisten, Betablockern, Diuretika, Aldosteronantagonisten und Herzglykosiden behandelt. Hauptrisikofaktor für die Entstehung von koronaren Herzkrankheiten, insbesondere von Herzinsuffizienz ist das metabolische Syndrom. Das metabolische Syndrom ist ein Krankheitsbild, dass sich aufgrund eines Lebensstils, der durch hyperkalorische Ernährung (permanente Überernährung) und einen Mangel an körperlicher Bewegung gekennzeichnet ist. Bei dem metabolischen Syndrom (auch tödliches Quartett, Reavan-Syndrom oder Syndrom X) treten kumulativ die vier Faktoren abdominale Adipositas, Bluthochdruck (arterielle Hypertonie), veränderte Blutfettwerte (Dyslipidämie) und Insulinresistenz (Typ-II-Diabetes) auf.

Es ist heute allgemein anerkannt, dass zwei Arten von Adipositas existieren. Die birnenförmige oder gynoide Fettverteilung steht für die Ansammlung von Fettgewebe im Hüft- und Schenkelbereich. Im Fall der apfelförmigen oder androiden Adipositas sammelt sich Fett in der Bauchhöhle an. Dieses viszerale Fett hat negative Merkmale; es sezerniert proinflammatorische Zytokine und Adipokine, wie TNFα, IL-6, Adiponectin, Leptin und andere (de Ferranti et al., 2008, Klein-Wieringa et al., 2011, Rai et al., 2011), die für die Entwicklung von Hypercholesterinämie, Arteriosklerose, Herzinfarkte und Typ-II-Diabetes verantwortlich sind. Diese Erkrankungen werden heutzutage als metabolisches Syndrom zusammengefasst (Ford et al., 2002, Watts et al., 2011).

Rheumatoide Osteoarthritis ist eine weit verbreitete Erkrankung, die aufgrund von autoimmunen Entzündungsprozessen auftritt (Hazes et al., 2011). Eine andere Arthritisart ist die früher Arthrose genannte Erkrankung, die vorwiegend Hüft- und Kniegelenke betrifft. Da sie vor allem bei übergewichtigen Menschen vorkommt, wurde angenommen, dass das Übergewicht der einzige Grund für den Knorpelverschleiß im Knie (Bliddal et al., 2011). In letzter Zeit haben sich Beweise dafür angesammelt, dass Fettkörper in Gelenken bei der Entstehung von Arthrose ebenfalls eine Rolle spielen (Gandhi et al., 2011, Klein-Wieringa et al., 2011, Rai et al., 2011). Diese Fettkörper, insbesondere der Hoffa-Fettkörper im Kniegelenk, sind bei übergewichtigen Menschen oftmals vergrößert (Hazes et al., 2011). Die Adipozyten in Gelenkfettkörpern haben mit den viszeralen Fettzellen die negativen Merkmale gemein; sie sezernieren proinflammatorische Adipozytokine und Zytokine und rufen somit in den Gelenken lokale Entzündungsprozesse hervor. Die sezernierten proinflammatorischen Zytokine hemmen die Reifung von Chondrozyten und bewirken die vermehrte Bildung von Matrixmetalloproteinasen, die die Infrastruktur von Gelenkknorpelgewebe zerstören (Rai et al., 2011). Diese Effekte werden in den Knie- und Hüftgelenken von dem Übergewicht adipöser Menschen verstärkt. Folglich ist Arthrose nicht nur eine degenerative Erkrankung, die von einer mechanischen Kompression von Knorpelgewebe herrührt; eine Entzündungskomponente ist bei der Entstehung und Weiterentwicklung dieser Erkrankung mindestens genauso wichtig. Aufgrund dieser Entzündungsprozesse, die auch im Knochen ablaufen, wird die Arthrose jetzt als Osteoarthritis bezeichnet, die bei vielen Patienten ein wesentlicher Bestandteil des metabolischen Syndroms ist.

Die Behandlung des metabolischen Syndroms erfolgt heute durch eine Umstellung des Lebensstils im Hinblick auf Ernährung und Bewegung mit dem Ziel das Körpergewicht und den Bauchumfang zu reduzieren, die Blutfette zu senken und das mögliche Auftreten des Typ-II-Diabetes möglichst weit hinauszuschieben. Es besteht deshalb ein Bedarf, neue Methoden und Arzneimittel zur Anwendung beim metabolischen Syndrom zur Verfügung zu stellen.

Daher besteht die Aufgabe der vorliegenden Erfindung darin, ein Arzneimittel zur Anwendung bei Sarkopenie, Herzinsuffizienz und zur Prävention bei Vorliegen der Risikofaktoren bereit zu stellen.

Überraschender Weise konnte gefunden werden, dass Cimicifuga, insbesondere Cimicifuga-Extrakte zur Anwendung bei der Behandlung und / oder Prophylaxe (Prävention, Vorbeugung) von Muskelschwäche, vorzugsweise Muskelschwäche der quergestreiften Muskulatur, insbesondere Sarkopenie (Schwäche der Muskeln im Alter) und Herzinsuffizienz (Herzmuskelschwäche) geeignet sind.

Daher betrifft die Erfindung ein Arzneimittel enthaltend Cimicifuga zur Anwendung bei Muskelschwäche oder um Muskelschwäche vorzubeugen, insbesondere Muskelschwäche der quergestreiften Muskulatur.

Eine Ausführungsform der Erfindung betrifft ein erfindungsgemäßes Arzneimittel wobei die Anwendung ausgewählt ist aus Sarkopenie, Herzmuskelschwäche.

Eine Ausführungsform der Erfindung betrifft ein erfindungsgemäßes Arzneimittel wobei die Anwendung ausgewählt ist aus metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle.

Eine Ausführungsform der Erfindung betrifft ein erfindungsgemäßes Arzneimittel zur Anwendung bei erhöhtem Spiegel von Leptin, Cholesterin, Glukose im Serum.

Eine Ausführungsform der Erfindung betrifft ein erfindungsgemäßes Arzneimittel wobei die Muskelschwäche ausgewählt ist aus einer Schwächung einer oder mehrerer Muskeln aus der Gruppe der Muskeln umfassend großen Brustmuskel, Deltamuskel, Bizeps, langer Hohlhandsehnenspanner, speicherseitiger Handbeuger, Oberarmspeichenmuskel, oberflächiger Fingerbeuger, Zwischenknochenmuskel, Trapezmuskel, Kopfwender, grader Bauchmuskel, vorderer Sägemuskel, mittlerer Gesäßmuskel, Spanner der Oberschenkelfascie, Kammmuskel, gerader Oberschenkelmuskel, äußerer Oberschenkelmuskel, Schneidermuskel, schlanker Muskel, langer Anzieher, vorderer Schienbeinmuskel, innerer Oberschenkelmuskel, innerer Wadenmuskel, kurzer Großzehenbeuger, Schollenmuskel, kurzer Zehenbeuger, langer Wadenbeinmuskel, äußerer schräger Bauchmuskel, oberflächliches Blatt, großer Rautenmuskel, großer Rundmuskel, dreiköpfiger Armstrecker, breitester Rückenmuskel, langer radialer Handstrecker, Fingerstrecker, Kleinfingerstrecker, ulnarer Handstrecker, großer Gesäßmuskel, äußerer Oberschenkelmuskel, schlanker Muskel, Plattsehnenmuskel, Halbsehnenmuskel, zweiköpfiger Oberschenkelmuskel, innerer Wadenmuskel, Schollenmuskel, Herzmuskel.

Eine Ausführungsform der Erfindung betrifft ein erfindungsgemäßes Arzneimittel wobei Cimicifuga Cimicifuga racemosa (CR) ist.

Eine Ausführungsform der Erfindung betrifft ein erfindungsgemäßes Arzneimittel wobei Cimicifuga ein Cimicifuga Extrakt, eine Fraktion eines Extrakts aus Cimicifuga, die Saponin-haltige Fraktion aus Cimicifuga, die Saponine aus Cimicifuga ist / sind.

Eine Ausführungsform der Erfindung betrifft ein erfindungsgemäßes Arzneimittel zur oralen oder subkutanen Applikation.

Eine weitere Ausführungsform der Erfindung betrifft eine Zusammensetzung, insbesondere pharmazeutische Zusammensetzung enthaltend Cimicifuga, Extrakt aus Cimicifuga, Saponin-haltige Fraktion aus Cimicifuga, Saponine aus Cimicifuga zur Anwendung bei Muskelschwäche oder um Muskelschwäche vorzubeugen.

Eine Ausführungsform der Erfindung betrifft eine erfindungsgemäße Zusammensetzung oder Arzneimittel in Form von Pulver, Granulat, Suppositorium, Lösung, Suspension, Paste, Gel, Creme, Lotion, Tablette, Dragee, insbesondere Manteltablette oder Calcium-Manteltablette oder in einer galenischen Formulierung, insbesondere einer oralen Formulierung wie Dragee, Tablette, Filmtablette, Kapsel, flüssige Verdünnung, insbesondere Tropfen, Saft, Sirup oder eine Formulierung zur topischen Anwendung wie Spray, Salbe, Emulsion, Puder, Pulver, flüssiges oder festes Präparat für die Inhalation, Kompressen, oder Lyophilisat samt geeigneter Zusatz und Hilfsstoffe.

Eine Ausführungsform der Erfindung betrifft eine Salbe, Saft, Lotion oder Zäpfchen enthaltend Cimicifuga, Extrakt aus Cimicifuga, Saponin-haltige Fraktion aus cimicifuga, Saponine aus Cimicifuga und gängige Hilfs- und Zusatzstoffe zur Anwendung bei Muskelschwäche und / oder zur Anwendung um Muskelschwäche vorzubeugen, insbesondere Muskelschwäche der quergestreiften Muskulatur, Sarkopenie, Herzmuskelschwäche, metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle und/ oder zur Anwendung bei erhöhtem Spiegel von Leptin, Cholesterin, Glukose im Serum.

Eine weitere Ausführungsform der Erfindung betrifft ein Nahrungsergänzungsmittel enthaltend Cimicifuga, insbesondere einen Extrakt aus Cimicifuga, die Saponin-haltige Fraktion aus Cimicifuga, die Saponine aus Cimicifuga zur Verwendung bei Muskelschwäche, insbesondere Muskelschwäche der quergestreiften Muskulatur, Sarkopenie, Herzmuskelschwäche, metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle, bei erhöhtem Spiegel von Leptin, Cholesterin, Glukose im Serum, Verlangsamung der Ganggeschwindigkeit, Verminderung der Beweglichkeit oder deren Vorbeugung.

Eine weitere Ausführungsform der Erfindung betrifft die Verwendung eines Nahrungsergänzungsmittels enthaltend Cimicifuga, insbesondere einen Extrakt aus Cimicifuga, die Saponin-haltige Fraktion aus Cimicifuga, die Saponine aus Cimicifuga bei Muskelschwäche, insbesondere Muskelschwäche der quergestreiften Muskulatur, Sarkopenie, Herzmuskelschwäche, metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle, bei erhöhtem Spiegel von Leptin, Cholesterin, Glukose im Serum, Verlangsamung der Ganggeschwindigkeit, Verminderung der Beweglichkeit oder zu deren Vorbeugung.

Eine Ausführungsform der Erfindung betrifft die erfindungsgemäße Verwendung von Cimicifuga, insbesondere eines Extrakts aus Cimicifuga, der Saponin-haltigen Fraktion aus Cimicifuga, der Saponine aus Cimicifuga zur Gewichtsreduktion, zur Vorbeugung gegen viszerales Fett, insbesondere bei Bewegungsmangel, hyperkalorischer Ernährung.

Eine weitere Ausführungsform der Erfindung betrifft ein Nahrungsmittel enthaltend Cimicifuga, einen Extrakt aus Cimicifuga, die Saponin-haltige Fraktion aus Cimicifuga, die Saponine aus Cimicifuga.

Die erfindungsgemäßen Anwendungen bzw. Verwendungen betreffen die Behandlung und Prophylaxe bei Menschen und Tieren, vorzugsweise bei Menschen.

Im Rahmen dieser Erfindung wird unter dem Begriff "Cimicifuga" (Traubensilberkerze) eine aus ursprünglich Nordamerika stammende (Arznei)Pflanze Cimicifuga racemosa (CR) - auch indianische Frauenwurzel genannt - verstanden, diese ist ca. 2 m groß und besitzt silbrig-weiße Blüten die lange Trauben bilden. Arzneilich verwendet wird der getrocknete Wurzelstock mit den Wurzeln (Droge). Erfindungsgemäß umfasst sind ebenfalls die folgenden Arten: C. foetida, C. dahurica, C. heracleifolia, C. simplex, C. japonica, C. europaea, C. acerina, C. elata, C. rubifolia. Weiterhin können Rhizome, Wurzeln, Stengel, Blätter und/oder Blütenblätter der Pflanzen verwendet werden.

Der Fachmann ist in der Lage aus Cimicifuga Pflanzenteile, insbesondere Wurzel, geeignete Extrakte herzustellen, wie bereits kommerziell erhältlich, z.B. Klimadynon® (supra). Z.B.

Auszüge oder Fraktionen können mittels organischer Lösungsmittel, wie Alkohole, insbesondere Ethanol, Dichlormethan, Heptan u.a. erhalten werden. Weiterhin sind wässrige Auszüge oder Fraktionen möglich. In der Literatur ist ein geeigneter Extrakt als "CR BNO 1055" beschrieben. Ferner können die Extrakte mit Super-Fluid-Extraktion (SFE) oder Super-Fluid-Chromatographie (SFC) erhalten werden (z.B. mittels überkritischen Gasen, wie CO₂ u.a.).

Zur Herstellung der erfindungsgemäßen Cimicifuga - Extrakte und wird zudem auf die technischen Lehren die Gegenstand von EP 1368605B1, EP 0753306B1 sind verwiesen.

Die Extrakte aus Cimicifuga sowie die Fraktionen können auch wie in Beispiel 2 beschrieben hergestellt werden. Insbesondere ist die Herstellung eines Extrakts aus Cimicifuga und von Fraktionen des Extrakts auch in Kapur et al., 2010b beschrieben.

Die Extrakte von Cimicifuga können in verschiedene Fraktionen getrennt werden, die sich in Ihren Inhaltsstoffen unterscheiden können. Erfindungsgemäß werden bevorzugt das Gesamtextrakt, die S-Fraktion (Saponin-haltige Fraktion) und die R-Fraktion (Rest-Fraktion) verwendet bzw. angewendet. Erfindungsgemäß können aber auch die Saponine aus Cimicifuga, seine unpolare und / oder seine polare Fraktion verwendet bzw. angewendet werden. Besonders bevorzugte Ausführungsformen der Erfindung betreffen die spezifische Anwendung bzw. Verwendung der S-Fraktion.

Die S-Fraktion des Cimicifuga Extrakts enthält Saponine (lat. Sapo = Seife). Saponine (auch Steroidsaponinen, Steroidalkaloidsaponinen, Triterpensaponinen, "Steroidale Glykoalkaloide") sind Glycoside von Steroiden, Steroidalkaloiden (z.B. stickstoffhaltige Steroide) oder Triterpenen. Saponine ergeben beim Schütteln mit Wasser oft einen seifenartigen Schaum und haben oft detergente Eigenschaften. Die S-Fraktion lässt sich deshalb beispielsweise als Schaumfraktion von der R-Fraktion abtrennen. Saponine bilden oft stabile Schäume, zeigen hämolytische Aktivität, beeinflussen die Membranpermeabilität, komplexieren Cholesterin, haben einen meist bitteren Geschmack, sind piscizid.

Erfindungsgemäß umfasst sind beispielsweise Cimicifuga Extrakte, S-Fraktion aus Cimicifuga Extrakt, die Aglycone (Sapogenine), beispielsweise Steroidsapogenine, Steroidalkaloidsapogenine, Tripensapogenine umfassen, beispielsweise Spirostane mit C-27-Grundgerüst, wie Spirostane (z.B. Digitogenin), Steroidalkaloidsapogenine mit C-27-Grundgerüst und Stickstoffatom, Triterpensapogenine mit C-30-Grundgerüst u. a. Cycloartana, Dammarane, Tirucallane, Lupane, Oleanane, Ursane, Taraxasterane.

Wirksame Inhaltsstoffe von Cimicifuga bzw. Cimicifuga-Extrakten und S- oder R-Fraktion können sein: Triterpene, wie Acetol- und Cimicigenolderivate, Polyphenole, wie Kaffeesäure und deren Derivate, Chlorogensäure, Piscidinsäure, Fucinolsäure, Ferula- und Isoferulasäure, Cimicifuginsäuren, Formononetin, Alkaloide u. a..

Im Rahmen dieser Erfindung wird unter "Muskelschwäche" die Rückbildung (Atrophie) und / oder Veränderung von Muskeln, Muskelgewebe, Muskelfasern, Muskelzellen, Muskulatur verstanden.

Muskulatur ist die Gesamtheit der Muskeln eines Menschen oder Tieres oder einzelner Organe (Organsysteme), beispielsweise Rückenmuskulatur. Ein Muskel ist ein kontraktiles Organ, das die Abfolge von Kontraktion und Erschlaffen innerer und äußerer Strukturen des Organismus bewegen kann, z.B. für Fortbewegung, Gestaltveränderung, Organfunktion. Das zugrunde liegende Gewebe ist das Muskelgewebe, das aus Muskelzellen bzw. Muskelfasern als wesentlichen Bestandteil umfasst bzw. daraus besteht.

"Muskelschwäche" umfasst beispielsweise die Abnahme (Verringerung) von Muskelmasse und / oder Veränderung des Muskels / Muskelgewebes, beispielsweise infolge der Verkleinerung des Durchmessers oder der Anzahl von Muskelfasern oder die Infiltration des Muskelgewebes mit anderen Zellen, insbesondere die Ansammlung von viszeralem Fett, Ablagerung von Fettzellen, Entstehen von Fettdepots und dergleichen. Die Muskelschwäche umfasst erfindungsgemäß bevorzugt Abnahme der Muskelmasse und / oder Veränderung der Muskeln / des Muskelgewebes der quergestreiften Muskulatur, wie Skelettmuskulatur und Herzmuskulatur. Eine besonders bevorzugte Ausführungsform der Erfindung betrifft die Veränderung der Muskulatur, des Muskelgewebes infolge der Einlagerung oder Ablagerung von Fettzellen, Fettdepots.

Bei der Skelettmuskulatur sind alle Muskeln erfindungsgemäß umfasst, die für aktive Körperbewegungen zuständig sind, beispielsweise auch die Muskeln der Zunge, des Kehlkopfs, des Zwerchfells, vorzugsweise die Muskeln des Rumpfes, der Beine und des Herzens. Die quergestreifte Muskulatur besteht aus lang gestreckten Zellen (Fasern) mit einer Vielzahl randständig unter der Zellmembran gelegenen elliptischen Kerne; die charakteristische Querstreifung beruht auf der Struktur der Myofibrillen (siehe z.B. Pschyrembel, de Gruyter, 263. Auflage, Berlin 2012). Die Muskeln umfassen die rote und die weiße Muskulatur. Umfasst sind beispielsweise die bereits vorstehend genannten Muskeln.

Der quergestreifte Herzmuskel (Myokard) besteht aus sich verzweigenden und anastomosierenden Herzmuskelzellen mit zentral gelegenem ovalem Zellkern. Dabei sind die mehr randständig gelagerten Myofibrillen sind weniger zahlreich als bei Skelettmuskelfasern. Eine Besonderheit der Herzmuskulatur sind die sog. Glanzstreifen (siehe z.B. Pschyrembl, de Gruyter, 263. Auflage, Berlin 2012). Eine besonders bevorzugte Ausführungsform der Erfindung betrifft die Veränderung der Herzmuskulatur, des Herzmuskelgewebes infolge der Einlagerung oder Ablagerung von Fettzellen, Fettdepots.

Folglich werden unter Muskelschwäche solche Veränderungen der Muskulatur, Muskeln, Muskelgewebe, Muskelzellen, Muskelfasern verstanden, die z.B. durch alterbedingte Veränderungen oder durch infektionsbedingte oder durch die Lebensgewohnheiten, beispielsweise Ernährungsgewohnheiten, Bewegungsgewohnheiten, bedingte Abnahme der Muskelmasse und / oder Veränderung von Muskeln, insbesondere durch die Ansammlung von Fett, viszeralem Fett, Fettzellen, Fettdepots in quergestreiften Muskeln hervorgerufen werden.

Sarkopenie ist beispielsweise die Abnahme der Muskelmasse im Alter bei gleichzeitiger Zunahme der Fettmasse verbunden mit einem steigenden Sturzrisiko (Def. nach Pschyrembl, de Gruyter 2012, Auflage 263, Berlin). Erfindungsgemäß umfasst ist die Verwendung bzw. Anwendung von Cimicifuga zur Behandlung und / oder Prävention von Sarkopenie.

Muskelschwäche, insbesondere Sarkopenie kann durch zunehmendes Alter, Bewegungsmangel, (Fehl-) Ernährung ausgelöst werden bzw. sich das Krankheitsbild der Sarkopenie etablieren. Für die Diagnose der Sarkopenie wird beispielsweise die Körperzusammensetzung und damit auch Zusammensetzung der Muskelmasse / des Muskelgewebes analysiert. Die Diagnose kann beispielsweise mit Hilfe der Dual-Röntgen-Absorptiometrie (DEXA) (Goldstandard) erfolgen. Eine andere häufig zur Diagnose der Sarkopenie angewendete Methode ist die bioelektrische Impendanzanalyse (BIA). Auch Computertomographie (CT) und Magnetresonanztomographie (MRT) gelten als zuverlässige Diagnostik.

Die Diagnose der Sarkopenie kann beispielsweise anhand der Definitionen (Kriterien) a) eines europäischen Expertengremiums gemäß A. J. Cruz-Jentoft et al. (European Working Group on Sarcopenia in Older People: Sarcopenia: European consensus on definition and diagnosis: Report of the European Working Group on Sarcopenia in Older People. In: Age Ageing 39, 2010, S. 412-423.) und / oder b) eines internationalen Expertengremiums gemäß M. Muscaritoli, et al. (Consensus definition of sarcopenia, cachexia and precachexia: joint document elaborated by Special Interest Groups (SIG) "cachexia-anorexia in chronic wasting diseases" and "nutrition in geriatrics". In: Clin Nutr 29, 2010, S. 154-159) erfolgen.

Erfindungsgemäß umfasst ist die Verwendung bzw. Anwendung bei der Diagnose von Sarkopenie bzw. Verdacht auf die Entstehung von Sarkopenie, beispielsweise aufgrund einer reduzierten Muskelmasse und / oder einer Verringerung der Ganggeschwindigkeit. Liegt die Muskelmasse des Probanden, die mit den genannten Methoden ermittelt wurde, zwei Standardabweichungen unterhalb des Mittelwertes einer gesunden jungen Referenzgruppe gleichen Geschlechts und gleichen ethnischen Hintergrundes und / oder besteht eine Verlangsamung der Ganggeschwindigkeit auf weniger als 0,8 m/s, kann Sarkopenie diagnostiziert werden (erniedrigte Muskelmasse und Verlangsamung der Ganggeschwindigkeit) bzw. besteht der Verdacht, dass sich Sarkopenie entwickelt (erniedrigte Muskelmasse oder Verlangsamung der Ganggeschwindigkeit).

Die Erfindung umfasst auch die Anwendung zur Behandlung von Muskelerkrankungen (Myopathien), insbesondere die Kardiomyphatie und Myopathien der quergestreiften Skelettmuskulatur. Leitsymptom aller Myopathien ist eine Schwäche der Muskulatur.

"Herzmuskelschwäche" (Herzinsuffizienz, Myokardinsuffizienz, myokardiale Dysfunktion) kann beispielsweise durch metabolisches Syndrom, insbesondere Fehlernährung, übermäßige Ernährung, fett- und / oder kalorienreiche Ernährung, Bewegungsmangel, Insulinresistenz, Typ 2 Diabetes (Diabetes mellitus), Hypertonie, Dylipidämie ausgelöst werden bzw. fortschreiten. Erfindungsgemäß umfasst sind Linksherzinsuffizienz, Rechtsherzinsuffizienz, Globalinsuffizienz, chronische Herzinsuffizienz. Erfindungsgemäß umfasst sind auch Kardiomyopathie, eine Gruppe von Erkrankungen, deren wesentliches Merkmal die direkte Beteiligung des Herzmuskels ist.

Die Diagnose der Herzmuskelschwäche kann beispielsweise mittels Echokardiographie, EKG, der laborschemischen Konzentration vom BNP (brain natriuretic peptide) und NTproBNP im Blut, Röntgen-Thorax-Aufnahme erfolgen. "Herzmuskelschwäche" umfasst erfindungsgemäß auch koronare Herzkrankheiten. Koronare Herzkrankheiten werden in den meisten Fällen durch Arteriosklerose ausgelöst. Hierbei bedingen Ablagerungen in den Gefäßwänden eine Versteifung sowie eine zunehmende Verminderung des Querschnitts in den Blutgefäßen. Daraus folgt eine Beeinträchtigung der Durchblutung und damit eine verminderte Sauerstoffversorgung der Herzmuskulatur (Missverhältnis zwischen Sauerstoffbedarf und Sauerstoffangebot) das zur Ischämie, Koronarinsuffizienz, Herzinsuffizienz führt.

Die koronare Herzkrankheit ist eine chronische Erkrankung, die im Verlauf von Jahren bis Jahrzehnten fortschreitet und bei der eine Heilung bisher nicht möglich ist. Hauptrisikofaktor für das Entstehen einer koronaren Herzerkrankung ist das metabolische Syndrom. Erfindungsgemäß umfasst ist deshalb insbesondere die Verwendung bei bzw. Anwendung zur Behandlung und / oder Prävention bei koronarer Herzkrankheit und / oder metabolischem Syndrom.

Das metabolische Syndrom kann nach den Kriterien der WHO (1988) folgendermaßen diagnostiziert werden. Metabolisches Syndrom liegt vor, wenn folgende Risikofaktoren bestehen: Diabetes mellitus (Typ-II-Diabetes), gestörte Glukosetoleranz, pathologischer Nüchternblutzucker bzw. Insulinresistenz, plus zwei der folgenden Parameter: Arterielle Hypertonie (Bluthochdruck; Blutdruck 140/90 mmHg), Dyslipidämie (Triglyceride > 1,695 mmol/L und HDL 0,9 mmol/L (bei Männern) bzw. 1,0 mmol/L (bei Frauen), abdominale Adipositas (= androide Adipositas) (Verhältnis von Taillen- zu Hüftumfang > 0,9 (bei Männern) bzw. > 0,85 (bei Frauen) und/oder ein BMI > 30 kg/m²).

Das metabolische Syndrom kann ebenfalls nach den Kriterien der IDF ("International Diabetes Foundation") aus 2005 bestimmt werden. Voraussetzung für das Vorhandensein des metabolischen Syndroms ist das Vorliegen einer abdominalen (auch bauchbetonte oder zentrale) Adipositas (Taillenumfang Männer 94 cm, bei Frauen 80 cm, kaukasischer Ethnie, für Asiaten gelten andere Werte) und noch mindestens zwei der Risikofaktoren: Nüchternblutzuckerwerte von > 100 mg/dl oder diagnostizierter Diabetes mellitus, Dyslipidämie (erhöhte Triglyceride > 150 mg/dl oder bereits eingeleitete Therapie zur Senkung der Triglyceride und niedriges HDL-Cholesterin (Männer < 40 mg/dl, Frauen < 50 mg/dl oder bereits eingeleitete Therapie zur Erhöhung des HDL)), Arterielle Hypertonie (Bluthochdruck ab > 130 mmHg systolisch und > 85 mmHg diastolisch oder bereits behandelte Hypertonie).

Das metabolische Syndrom kann auch nach den Kriterien der NCEP-ATP-III ("National Expert Panel on Detection, Evaluation, and Treatment of High Blood Cholesterol in Adults") aus 2001 bestimmt werden. Danach wird die Diagnose metabolisches Syndrom gestellt, wenn mindestens drei der folgenden fünf Kriterien erfüllt sind: Abdominale Adipositas (bestimmt durch einen Bauchumfang von über 102 cm bei Männern oder über 88 cm bei Frauen), Triglyzeride von über 150 mg/dL, HDL-Cholesterin von unter 40 mg/dL bei Männern bzw. < 50 mg/dL bei Frauen, Bluthochdruck (130/85 mmHg oder mehr), Nüchternblutzucker von über 110 mg/dL (oder Vorliegen von Diabetes Typ 2).

Ein Risiko für das metabolische Syndrom bzw. das Vorliegen eines metabolischen Syndroms kann auch alleine aufgrund des Taillenumfangs - Taillenumfang größer 88 cm (Frauen) bzw. 102 cm (Männer) - diagnostiziert werden.

Die Erfindung betrifft deshalb auch die Verwendung bzw. Anwendung zur Prävention, Behandlung bei Vorliegen von Risikofaktoren, wie beispielsweise den Risikofaktoren eines Fettverteilungsmusters mit Fettdepots im Bauchraum und an den inneren Organen (androide Adipositas, abdomiale Adipositas, inneres Bauchfett, "intraabdominales Fett", viszerales Fett), beispielsweise bei einem Taillenumfang größer 88 cm (Frauen) bzw. 102 cm (Männer), bei metabolischem Syndrom diagnostiziert nach WHO, IDF und / oder NCEP-ATP-III.

Daher betrifft die Erfindung auch die Verwendung / Anwendung bei kardiometabolischen Risikofaktoren, beispielsweise im Rahmen einer Präventionsbehandlung, insbesondere bei (abdominaler, androider) Adipositas, Diabetes mellitus, Fettstoffwechselstörungen (Triglyceride, HDL-Cholerstin), Bluthochdruck, metabolischem Syndrom, Dyslipidämie u.a..

Die Erfindung betrifft auch ein Arzneimittel (Wirkstoff, Formulierung)/ Zubereitung (Pharmazeutische Zubereitung) / Nahrungsmittel (Nahrungsergänzungsmittel) zur Anwendung bei Vorliegen der vorgenannten Indikationen oder Risikofaktoren. Alle vorstehenden Indikationen sind z.B. im Pschyrembel, de Gruyter, 263. Auflage, Berlin 2012 beschrieben.

Das erfindungsgemäße Arzneimittel umfasst Cimicifuga, vorzugsweise Cimicifuga racemosa, insbesondere Extrakte aus Cimicifuga oder Fraktionen des Extrakts aus Cimicifuga (S-Fraktion) als Wirkstoff.

Ferner betrifft die Erfindung daher eine pharmazeutische Formulierung enthaltend ein erfindungsgemäßes Arzneimittel (auch "Wirkstoff" oder "Zusammensetzung" genannt).

Beispiele für besonders geeignete pharmakologisch verträgliche Träger sind dem Fachmann bekannt und umfassen gepufferte Kochsalzlösungen, Wasser, Emulsionen wie z.B. ÖI/Wasser-Emulsionen, verschiedene Arten von Detergentien, sterile Lösungen, etc.

Pharmazeutische Zusammensetzungen, die solche Träger umfassen, können mittels bekannter konventioneller Methoden formuliert werden. Diese pharmazeutischen Zusammensetzungen können einem Individuum / Patienten in einer geeigneten Dosis verabreicht werden. Die Verabreichung kann oral, parenteral, intravenös, intraperitoneal, subcutan, intramuskulär, lokal, intranasal, oder intradermal, oder über einen Katheter an einer Stelle in einer Arterie erfolgen. Die Art der Dosierung wird vom behandelnden Arzt entsprechend den klinischen Faktoren bestimmt.

Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden.

Die erfindungsgemäßen Arzneimittel können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Formulierungen seien Tabletten, Dragees, Kapseln, Pillen, Granulate, Suppositorien, Lösungen, Saft, Suspensionen und Emulsionen, Pasten, Salben, Gele, Cremes, Lotions, Puder und (Nasen)Sprays genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können. Weiterhin ist bevorzugt, dass die galenische Form eine Manteltablette, insbesondere eine Calcium-Manteltablette ist, wie in W02002100422 offenbart.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant- Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die erfindungsgemäßen pharmazeutischen Zusammensetzungen oder Zubereitungen können in Form von Dosierungseinheiten hergestellt werden. Dies bedeutet, dass die Zusammensetzungen in Form einzelner Teile, vorzugsweise Kapseln, Dragees und Ampullen vorliegen, deren Wirkstoffgehalt an dem erfindungsgemäßen Extrakt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge des erfindungsgemäßen Extrakts (Wirkstoff), die bei einer Applikation verabreicht wird und die gewöhnlich einer ganzen, einer halben, einem Drittel oder einem Viertel einer Tagesdosis entspricht.

In einer weiteren bevorzugten Ausführungsform kann die galenische Formulierung einer Calziummanteltablette gewählt werden, wie in EP1392337 offenbart.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können. Das erfindungsgemäße Nahrungsergänzungsmittel kann ebenfalls in Form einer Zusammensetzung verabreicht werden, wie oben beschrieben. In einer weiteren Ausführungsform kann das Nahrungsergänzungsmittel einem beliebigen Nahrungsmittel oder Lebensmittel, einschließlich Wasser, zugesetzt werden. Nahrungs- und Lebensmittel im Sinne dieser Erfindung sind insbesondere solche, wie nicht abschließend in EU-Richtlinie 2002/46/EG vom 10. Juni 2002 definiert.

Die Erfindung betrifft auch die Verfahren zur Herstellung der erfindungsgemäßen Arzneimittel, Zusammensetzungen, pharmazeutischen Zusammensetzungen, Nahrungsmittel, Nahrungsergänzungsmittel.

Nachfolgende Beispiele und Figuren dienen zur Erläuterung der Erfindung ohne die Erfindung auf diese Beispiele einzuschränken.

Figuren
Figur 1: Figur 1 zeigt die Muskelfläche im oberen Anteil des Unterschenkels von ovariektomierten Ratten, welche mit CR BNO 1005, der Saponin (S)-Fraktion oder der Rest (R)-Fraktion behandelt wurden oder die unbehandelt (Ko) blieben.
Figur 2: Figur 2 zeigt die höheren Querschnitte der Muskelfasern (Muskelfaserfläche) im oberen Anteil des Unterschenkels von ovariektomierten Ratten, welche mit CR BNO 1055, der Saponin-Fraktion (S-Fraktion) oder Rest-Fraktion (R-Fraktion) behandelt worden waren.
Figur 3: Histologische Präparation des Knieglenks.
Figur 4: Effekt von CR BNO 1055 und der S- und R-Fraktionen auf die Größe des paratibialen Fettdepots (PFD) (a), Serum Leptin Levels (b). Serum Leptin als Funktion der Größe des PFD korrelierte Signifikanz (c).
Figur 5: Effekte von CR BNO 1055 und seiner S- und R-Fraktionen auf das Serum Cholesterol (a) das signifikant mit der Größe des PFD korreliert (b). Die Effekte des Extrakts auf die Extrakte auf Serum Glykose sind ebenfalls gezeigt (c).
Figur 6: Effekte von CR BNO 1055 und seiner S- and R-Fraktionen auf die Größe der Hoffa's Fettdepots im Knie und Kniegelenk (a) und auf die Dicke (b). Die Dicke als Funktion der Größe des Fettdepots (c).

### Beispiele

Ovariektomierte (OVX) Ratten werden adipös und entwickeln ein metabolisches Syndrom, einschließlich Hyperglykämie (Chen et al., 2001, Gorres et al., 2011, Seidlova-Wuttke et al., 2003, Zoth et al., 2010). Darüber wirkt sich eine OVX durch Ausdünnen der Knorpelschicht nachteilig auf das Gelenkknorpelgewebe auswirkt. Deshalb sind ovariektomierte Ratten ein gutes Modell für die Untersuchung des metabolischen Syndroms und der Osteoarthritis (Kapur et al., 2010a).

### Beispiel 1:

Die ovx Ratten erhalten frühestens 14 Tage nach der Ovariektomie (ovx) die jeweilige Prüfsubstanz (hier: Cimicifuga Extrakt BNO 1055) oder Fraktionen von Cimicifuga Extrakt. Es wurde der Effekt der (S)-Fraktion (Saponin-haltige Fraktion), der (R )-Fraktion (Rest-Fraktion) und des BNO 1055 Gesamtextrakts auf die Muskeln der Ratten untersucht. Die Verabreichung erfolgt über 28 Tage mit dem Futter. Die Fraktionen (C001 = Gesamtextrakt; C001/S = S-Fraktion; C001/R = Restfraktion) waren jeweils in Cremophor gelöst, die Kontrolltiere enthielten das Vehikel (Ko). Die Muskeln (Muskelfasern) wurden nach vier Wochen untersucht bzw. vermessen.

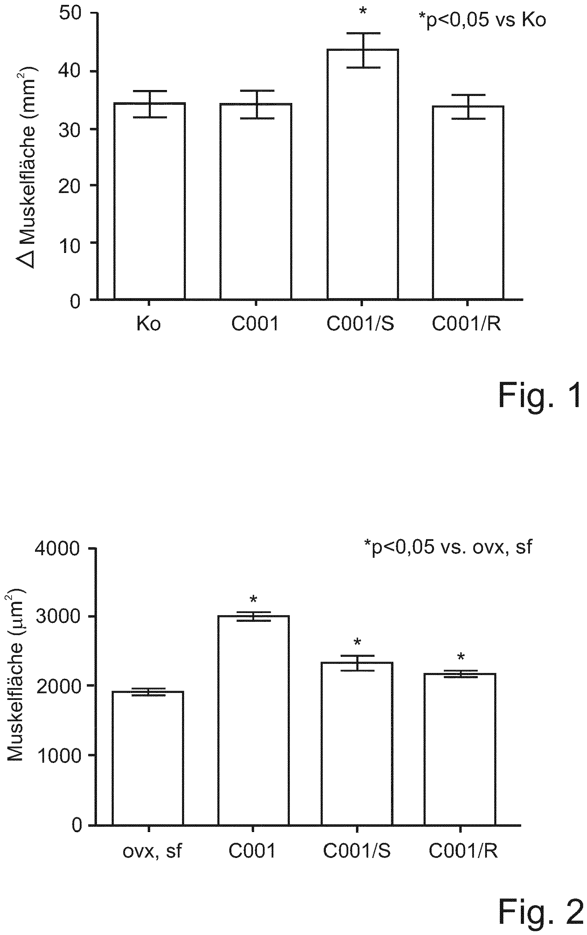

Die Untersuchung der Muskelfläche im oberen Anteil des Unterschenkels erfolgte mittels Computertomographie. Die Muskelfläche von M. gastrocnemius war in den mit S-Fraktion behandelten Muskeln bzw. Tieren deutlich erhöht, d.h. es war mehr Muskulatur vorhanden (Figur 1).

Das ist vermutlich auf die in Figur 2 gezeigten deutlich höheren Querschnitte der Muskelfasern zurückzuführen. Das spricht für eine deutliche Zunahme der Kraft der Muskulatur durch Cimicifuga.

Außerdem wurden die Querschnitte der Muskelfasern der Herzen der Ratten ebenfalls untersucht. Es zeigte sich, dass auch die Querschnitte der Muskelfasern des Herzens deutlich größer sind als bei den Ko-Tieren. Auch das spricht für die Zunahme der Kraft des Herzens durch Cimicifuga.

### Beispiel 2

Nach 4 Wochen Eingewöhnung an die Tierhaltungsanlage wurden die 270 ± 4,4 g wiegenden Ratten ovariektomiert und die Gruppen wurden entsprechend den Körpergewichten (KG) randomisiert, so dass jede Gruppe identische KG aufwies. Die Ratten erhielten sojafreies Futter (V 1355 R-Z, 10 mm, arm an Phytoöstrogenen, Ssniff, Soest, Deutschland), um die Exponierung mit von Soja stammenden Östrogenverbindungen auszuschließen, die in normalem Rattenfutter vorzufinden sind. Eine isokalorische Proteinsupplementation wurde durch zugesetzte Kartoffelproteine sichergestellt. Die Testsubstanzen waren der CR-Spezialextrakt CR BNO 1055 und die daraus fraktionierten lipophilen und hydrophilen Verbindungen.

### Pflanzliches Material:

Als pflanzliches Material wurde der Spezialextrakt von *Cimicifuga racemosa,* CR BNO 1055 verwendet, dessen Herstellung bereits beschrieben wurde (Kapur et al., 2010b).

### Fraktionierungsprozess:

Die Trennung der Triterpenglycoside von den hydrophilen Bestandteilen des Spezialextrakts BNO 1055 wurde mittels Flüssig-Flüssig-Extraktion (FFE) erzielt. Der Rohextrakt wurde zum Teil in Wasser gelöst und für 15 Minuten beschallt. Zur besseren Trennung der Phenylpropanoide wurde der pH-Wert der Suspension auf 8,5 eingestellt, Dichlormethan wurde zugegeben und es wurde für 1 h bei 33 °C gemischt. Das Gemisch wurde in einen Scheidetrichter überführt, um die lipophile Phase von der hydrophilen Phase zu trennen. Nach Aufteilen der Dichlormethanphase wurde die FFE wiederholt.

Der Originalextrakt CR BNO 1055 und die daraus hergestellten S- und R-Fraktionen wurden mit dem oben beschriebenen Futter gemischt. Die Tiergruppen, ihre tägliche Futter- und Extraktaufnahme und die Gewichtszunahme vom Beginn bis zum Ende des 4 Wochen dauernden Versuchszeitraums sind in Tabelle 1 detailliert aufgeführt. Zum Ende dieses Zeitraums wurden die Tiere unter CO₂-Narkose geopfert, vom Rumpf wurde Blut abgenommen und eine Probe, die aus dem unteren Teil des Femurs, dem Kniegelenk und dem oberen Teil der Tibia bestand, wurde von jedem Tier genommen.

**Tabelle 1: Zusammensetzung des Futters**

| **Gruppe** | **Anzahl der Tiere (n)** | **Futteraufnahme (g/Tag/ Tier)** | **Aufnahme der Testsubstanz (mg/Tier/Tag)** | **Δ BW (g) Zunahme im Bezug auf das Anfangsgewicht (BW)** |
|---|---|---|---|---|
| Co (ovx) | 10 | 17 | keine | 94.48 |
| ovx CR BNO 1055 | 10 | 16.45 | 8.22 | 74.41* |
| ovx S-Fraktion | 10 | 17.42 | 2.05 | 87.47 |
| ovx R-Fraktion | 10 | 18.49 | 7.07 | 81.94 |

| | | | | |
|---|---|---|---|---|
| *p < 0.05 vs Co (ovx) | | | | |

### Quantitative Computertomographie (qCT)

Vor der OVX und einen Tag vor der Opferung der Tiere wurden die oberen Metaphysen der Tibia einer qCT unter Verwendung des Instruments Stratec x CT 5.40 (Stratec Inc., Pforzheim, Deutschland) unterzogen. Der Scanner wurde unter Anwendung einer Isofluran-Narkose auf Höhe der epiphysären Wachstumszone angeordnet, wobei es sich um die Bezugslinie handelte. In einem Abstand von 3,75 und 4,25 mm distal zu dieser Bezugslinie wurden qCT-Messungen durchgeführt. Die Dichten werden als Mittelwert der 1. und der 2. CT-Ebene berechnet und werden in mg/cm³ angegeben. Bereiche mit Dichten unter 40 mg/cm³ werden als Fettgewebe angesehen. Dies ermöglichte die Bestimmung eines Fettdepots, das sich im unteren Hinterbein befindet (paratibiales Fettdepot (PFD)), dass es bei ovariektomierten Tieren groß und bei intakten oder mit OVX-E2 behandelten Tieren viel kleiner ist (Seidlova-Wuttke et al., 2010). Somit hatte dieses Fettdepot dieselben Eigenschaften wie das viszerale Fettgewebe.

### Histologische Beurteilung des Kniegelenks

Zum Ende des Versuchszeitraums wurden jedem Tier verschiedene Organe entnommen, u. a. Proben des unteren Femurs, des unteren Kniegelenks und der oberen Tibia.

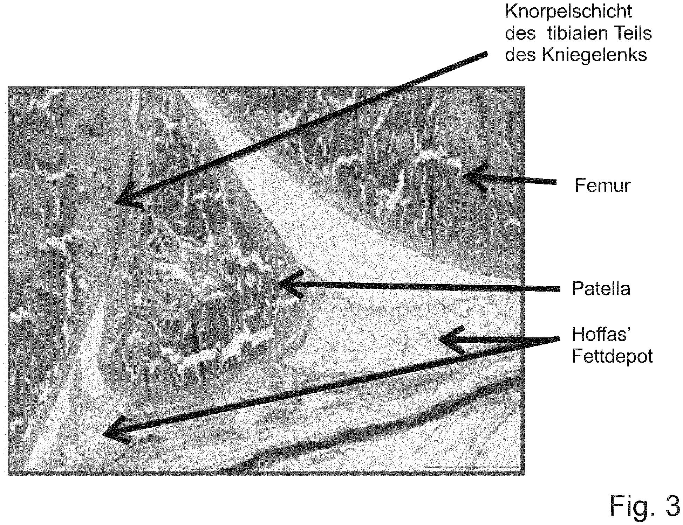

Die gesäuberten Knochen-/Gelenkproben wurden Epoxidharz eingebettet und über einen Zeitraum von 4 - 6 Wochen aushärten gelassen. Nach dem Aushärten wurden Längsschnitte mit einer Dicke von 5 µm hergestellt und nach Goldner gefärbt. In Schnitten der Mittensektion (Figur 3) (10/Tier) wurde die Oberfläche des Hoffa-Fettkörpers perimetrisch bestimmt und die Dicke der Knorpelschicht des Tibiateils des Kniegelenks wurde quantifiziert.

### Serumanalyse

Die Serumleptinspiegel wurden mit einem im Handel erhältlichen Radioimmunassay (DSL Inc., Webster, Texas, USA) gemessen. Serumglukose und -cholesterin wurden mit einem Analysegerät Hitachi 911 (Roche, Mannheim, Deutschland) quantifiziert.

### Statistische Analyse

Die histologischen Parameter wurden mithilfe eines Computerprogramms (Olympus-Bildanalysesoftware Analysis, Version Cell Vision, Hamburg, Deutschland) quantifiziert. Alle Daten werden als arithmetisches Mittel ± Standardfehler des Mittelwerts (SEM) dargestellt. Es wurden eine einfache ANOVA, gefolgt von einem Dunnett-Post-hoc-Test für Mehrfachvergleiche durchgeführt, um die Unterschiede zwischen den Kontrollen (OVX) und den Behandlungsgruppen (Prism™, Graph Pad, San Diego, USA) zu vergleichen. P-Werte von < 0,05 wurden als statistisch signifikant angesehen. Mit demselben Programm wurden Korrelationsstatistiken durchgeführt.

### Ergebnisse

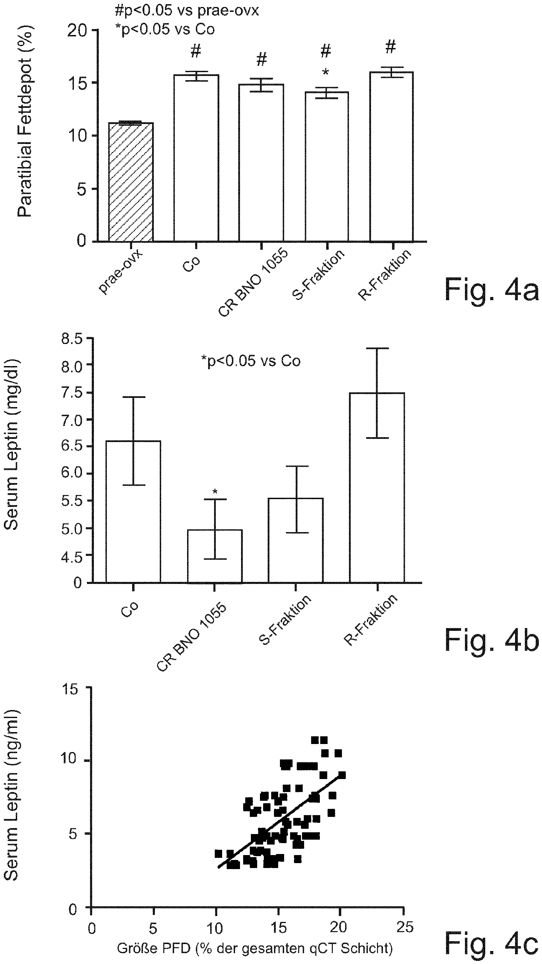

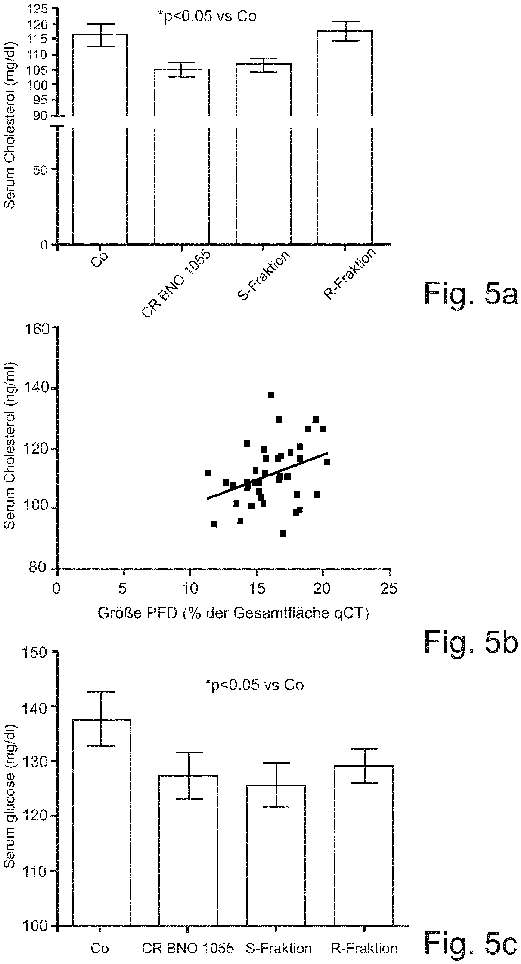

Tabelle 1 führt detailliert die Zusammensetzung des Futters, die Futteraufnahme und die daraus berechnete Aufnahme der Testextrakte sowie die Endkörpergewichte der Tiere auf. Die höchsten Körpergewichte lagen bei den ovariektomierten Tieren vor und signifikant niedrigere Werte wurden bei den mit CNR BNO 1055 und der S-Fraktion behandelten ovariektomierten Ratten festgestellt. Figur 4 zeigt die Größe des paratibialen Fettdepots (PFD) (Figur 4a) und die Serumleptinspiegel (Figur 4b), die bei den ovariektomierten Tieren am größten bzw. höchsten und bei den mit BNO 1055 und der S-Fraktion behandelten ovariektomierten Ratten signifikant kleiner bzw. niedriger waren. Die Größe des PFD korrelierte signifikant mit den Serumleptinspiegeln (Figur 4c). Die hohen Serumcholesterinwerte bei den ovariektomierten Ratten wurden ebenfalls von CR BNO 1055 und der S-Fraktion gesenkt (Figur 5a) und das Serumcholesterin korrelierte ebenso signifikant mit der Größe des PFD (Figur 5b). Figur 5c zeigt detailliert, dass die hohen Serumglukosespiegel bei den ovariektomierten Tieren von CR BNO 1055 und der daraus hergestellten S-Fraktion signifikant gesenkt wurden. Die R-Fraktion hatte keine signifikante Auswirkung auf die Serumglukose.

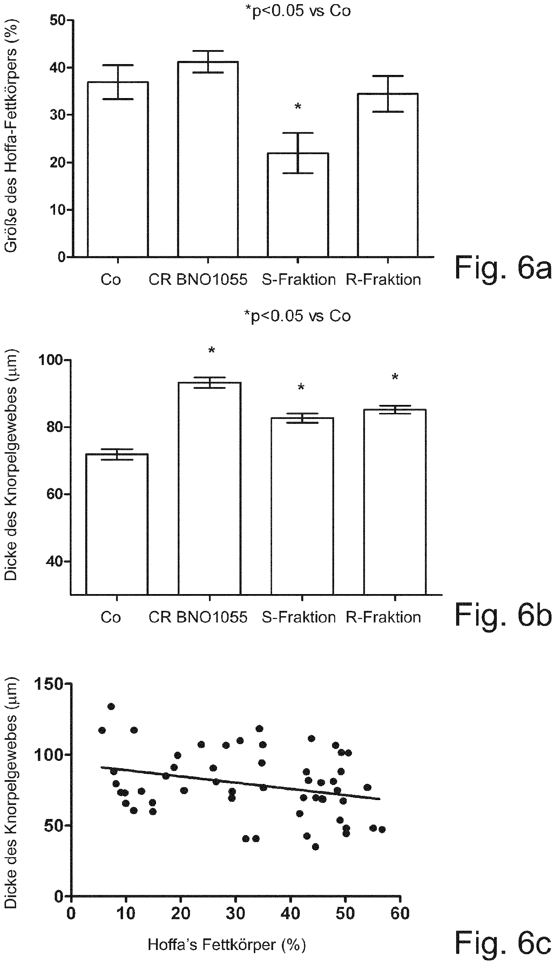

Die Größe des Hoffa-Fettkörpers ist in Figur 6a gezeigt. Große Fettkörper im Kniegelenk lagen bei den ovariektomierten Tieren vor und waren bei den mit der S-Fraktion behandelten Tieren signifikant kleiner. Figur 6b zeigt detailliert die Dicke des Knorpelgewebes des Tibiateils des Kniegelenks, die bei den ovariektomierten Tieren am geringsten und bei den mit BNO 1055 und der S- und der R-Fraktion behandelten Tieren signifikant größer war. Die Größe des Hoffa-Fettkörpers korrelierte umgekehrt mit der Höhe des Knorpelgewebes Figur 6c.

### Literaturzitate

Bliddal H., Leeds A.R., Stigsgaard L., Astrup A., Christensen R., 2011. Weight loss as treatment for knee osteoarthritis symptoms in obese patients: 1-year results from a randomised controlled trial. Ann Rheum Dis. 70, 1798-1803.
Chamberlain G., Fox J., Ashton B., Middleton J., 2007. Concise review: mesenchymal stem cells: their phenotype, differentiation capacity, immunological features, and potential for homing. Stem Cells. 25, 2739-2749.
Chen Y., Heiman M.L., 2001. Increased weight gain after ovariectomy is not a consequence of leptin resistance. Am J Physiol Endocrinol Metab. 280, E315-322.
de Ferranti S., Mozaffarian D., 2008. The perfect storm: obesity, adipocyte dysfunction, and metabolic consequences. Clin Chem. 54, 945-955.
Espinola-Klein C., Gori T., Blankenberg S., Munzel T., 2011. Inflammatory markers and cardiovascular risk in the metabolic syndrome. Front Biosci. 16, 1663-1674.
Ford E.S., Giles W.H., Dietz W.H., 2002. Prevalence of the metabolic syndrome among US adults: findings from the third National Health and Nutrition Examination Survey. JAMA. 287, 356-359.
Gandhi R., Takahashi M., Virtanen C., Syed K., Davey J.R., Mahomed N.N., 2011. Microarray analysis of the infrapatellar fat pad in knee osteoarthritis: relationship with joint inflammation. J Rheumatol. 38, 1966-1972.
Gorres B.K., Bomhoff G.L., Gupte A.A., Geiger P.C., 2011. Altered estrogen receptor expression in skeletal muscle and adipose tissue of female rats fed a high-fat diet. J Appl Physiol. 110, 1046-1053.
Hazes J.M., Luime J.J., 2011. The epidemiology of early inflammatory arthritis. Nat Rev Rheumatol. 7, 381-390.
Jarry H., Metten M., Spengler B., Christoffel V., Wuttke W., 2003. In vitro effects of the Cimicifuga racemosa extract BNO 1055. Maturitas. 44 Suppl 1, S31-38. 14
Kapur P., Wuttke W., Jarry H., Seidlova-Wuttke D., 2010a. Beneficial effects of beta- Ecdysone on the joint, epiphyseal cartilage tissue and trabecular bone in ovariectomized rats. Phytomedicine. 17, 350-355.
Kapur P., Wuttke W., Seidlova-Wuttke D., 2010b. The Cimicifuga racemosa special extract BNO 1055 prevents hot flashes in ovariectomized rats. Phytomedicine. 17, 890-894.
Kawai M., Sousa K.M., MacDougald O.A., Rosen C.J., 2010. The many facets of PPARgamma: novel insights for the skeleton. Am J Physiol Endocrinol Metab. 299, E3-9. Klein-Wieringa I.R., Kloppenburg M., Bastiaansen-Jenniskens Y.M., Yusuf E., Kwekkeboom J.C., El-Bannoudi H., Nelissen R.G., Zuurmond A., Stojanovic-Susulic V., Van Osch G.J., Toes R.E., Ioan-Facsinay A., 2011. The infrapatellar fat pad of patients with osteoarthritis has an inflammatory phenotype. Ann Rheum Dis. 70, 851-857.
Koppen A., Kalkhoven E., 2010. Brown vs white adipocytes: the PPARgamma coregulator story. FEBS Lett. 584, 3250-3259. Othman R.A., Moghadasian M.H., 2011. Beyond cholesterollowering effects of plant sterols: clinical and experimental evidence of anti-inflammatory properties. Nutr Rev. 69, 371-382. Rai M.F., Sandell L., 2011. Inflammatory mediators: tracing links between obesity and osteoarthritis. Crit Rev Eukaryot Gene Expr. 21, 131-142.
Saggini A., Anogeianaki A., Maccauro G., Tete S., Salini V., Caraffa A., Conti F., Fulcheri M., Galzio R., Shaik-Dasthagirisaheb Y.B., 2011. Cholesterol, cytokines and diseases. Int J Immunopathol Pharmacol. 24, 567-581. Seidlova-Wuttke D., Jarry H., Becker T., Christoffel V., Wuttke W., 2003. Pharmacology of Cimicifuga racemosa extract BNO 1055 in rats: bone, fat and uterus. Maturitas. 44 Suppl 1, S39-50.
Takada I., Kouzmenko A.P., Kato S., 2009. Molecular switching of osteoblastogenesis versus adipogenesis: implications for targeted therapies. Expert Opin Ther Targets. 13, 593- 603. 15
Watts G.F., Karpe F., 2011. Republished review: Triglycerides and atherogenic dyslipidaemia: extending treatment beyond statins in the high-risk cardiovascular patient. Postgrad Med J. 87, 776-782.
Zoth N., Weigt C., Laudenbach-Leschowski U., Diel P., 2010. Physical activity and estrogen treatment reduce visceral body fat and serum levels of leptin in an additive manner in a diet induced animal model of obesity. J Steroid Biochem Mol Biol. 122, 100-105.

## Patentansprüche

1. Arzneimittel enthaltend Cimicifuga zur Anwendung bei Muskelschwäche oder um Muskelschwäche vorzubeugen, insbesondere Muskelschwäche der quergestreiften Muskulatur.

2. Arzneimittel nach Anspruch 1 wobei die Anwendung ausgewählt ist aus Sarkopenie, Herzmuskelschwäche.

3. Arzneimittel nach einem der vorgehenden Ansprüche wobei die Anwendung ausgewählt ist aus metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle.

4. Arzneimittel nach einem der vorgehenden Ansprüche zur Anwendung bei erhöhtem Spiegel von Leptin, Cholesterin, Glukose im Serum.

5. Arzneimittel nach einem der vorgehenden Ansprüche wobei die Muskelschwäche ausgewählt ist aus einer Schwächung einer oder mehrerer Muskeln aus der Gruppe der Muskeln umfassend großen Brustmuskel, Deltamuskel, Bizeps, langer Hohlhandsehnenspanner, speicherseitiger Handbeuger, Oberarmspeichenmuskel, oberflächiger Fingerbeuger, Zwischenknochenmuskel, Trapezmuskel, Kopfwender, grader Bauchmuskel, vorderer Sägemuskel, mittlerer Gesäßmuskel, Spanner der Oberschenkelfascie, Kammmuskel, gerader Oberschenkelmuskel, äußerer Oberschenkelmuskel, Schneidermuskel, schlanker Muskel, langer Anzieher, vorderer Schienbeinmuskel, innerer Oberschenkelmuskel, innerer Wadenmuskel, kurzer Großzehenbeuger, Schollenmuskel, kurzer Zehenbeuger, langer Wadenbeinmuskel, äußerer schräger Bauchmuskel, oberflächliches Blatt, großer Rautenmuskel, großer Rundmuskel, dreiköpfiger Armstrecker, breitester Rückenmuskel, langer radialer Handstrecker, Fingerstrecker, Kleinfingerstrecker, ulnarer Handstrecker, großer Gesäßmuskel, äußerer Oberschenkelmuskel, schlanker Muskel, Plattsehnenmuskel, Halbsehnenmuskel, zweiköpfiger Oberschenkelmuskel, innerer Wadenmuskel, Schollenmuskel, Herzmuskel.

6. Arzneimittel nach einem der vorgehenden Ansprüche wobei Cimicifuga Cimicifuga racemosa (CR) ist.

7. Arzneimittel nach einem der vorgehenden Ansprüche wobei Cimicifuga ein Cimicifuga Extrakt, eine Fraktion eines Extrakts aus Cimicifuga, die Saponin-haltige Fraktion aus Cimicifuga, die Saponine aus Cimicifuga ist / sind.

8. Arzneimittel nach einem der vorgehenden Ansprüche zur oralen oder subkutanen Applikation.

9. Zusammensetzung, insbesondere pharmazeutische Zusammensetzung enthaltend Cimicifuga, Extrakt aus Cimicifuga, Saponin-haltige Fraktion aus Cimicifuga, Saponine aus Cimicifuga zur Anwendung bei Muskelschwäche oder um Muskelschwäche vorzubeugen.

10. Zusammensetzung oder Arzneimittel nach einem der vorgehenden Ansprüche in Form von Pulver, Granulat, Suppositorium, Lösung, Suspension, Paste, Gel, Creme, Lotion, Tablette, Dragee, insbesondere Manteltablette oder Calcium-Manteltablette oder in einer galenischen Formulierung, insbesondere einer oralen Formulierung wie Dragee, Tablette, Filmtablette, Kapsel, flüssige Verdünnung, insbesondere Tropfen, Saft, Sirup oder eine Formulierung zur topischen Anwendung wie Spray, Salbe, Emulsion, Puder, Pulver, flüssiges oder festes Präparat für die Inhalation, Kompressen, oder Lyophilisat samt geeigneter Zusatz und Hilfsstoffe.

11. Salbe, Saft, Lotion oder Zäpfchen enthaltend Cimicifuga, Extrakt aus Cimicifuga, Saponin-haltige Fraktion aus Cimicifuga, Saponine aus Cimicifuga und gängige Hilfs- und Zusatzstoffe zur Anwendung bei Muskelschwäche und / oder zur Anwendung um Muskelschwäche vorzubeugen, insbesondere Muskelschwäche der quergestreiften Muskulatur, Sarkopenie, Herzmuskelschwäche, metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle, bei erhöhtem Spiegel von Leptin, Cholesterin, Glukose im Serum.

12. Nahrungsergänzungsmittel enthaltend Cimicifuga, insbesondere einen Extrakt aus Cimicifuga, die Saponin-haltige Fraktion aus Cimicifuga, die Saponine aus Cimicifuga zur Verwendung bei Muskelschwäche, insbesondere Muskelschwäche der quergestreiften Muskulatur, Sarkopenie, Herzmuskelschwäche, metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle, bei erhöhtem Spiegel von Leptin, Cholesterin, Glukose im Serum, Verlangsamung der Ganggeschwindigkeit, Verminderung der Beweglichkeit oder deren Vorbeugung.

13. Verwendung eines Nahrungsergänzungsmittels enthaltend Cimicifuga, insbesondere einen Extrakt aus Cimicifuga, die Saponin-haltige Fraktion aus Cimicifuga, die Saponine aus Cimicifuga bei Muskelschwäche, insbesondere Muskelschwäche der quergestreiften Muskulatur, Sarkopenie, Herzmuskelschwäche, metabolischem Syndrom, Hypercholesterinämie, Arteriosklerose, Herzinfarkt, Typ-II-Diabetes, Adipositas, insbesondere androider Adipositas, viszeralem Fett, insbesondere viszeralem Fett im Gelenk, im Muskel, in der Bauchhöhle, bei erhöhtem Spiegel von Leptin, Cholesterin, Glukose im Serum, Verlangsamung der Ganggeschwindigkeit, Verminderung der Beweglichkeit oder zu deren Vorbeugung.

14. Verwendung von Cimicifuga, insbesondere eines Extrakts aus Cimicifuga, der Saponin-haltigen Fraktion aus Cimicifuga, der Saponine aus Cimicifuga zur Gewichtsreduktion, zur Vorbeugung gegen viszerales Fett, insbesondere bei Bewegungsmangel, hyperkalorischer Ernährung.

15. Nahrungsmittel enthaltend Cimicifuga, einen Extrakt aus Cimicifuga, die Saponin-haltige Fraktion aus Cimicifuga, die Saponine aus Cimicifuga.
